# EUROPEAN PATENT SPECIFICATION

(11) **EP 2 016 886 B1**
(45) Date of publication and mention of the grant of the patent: **13.04.2011**
(21) Application number: 08012790.5
(22) Date of filing: 15.07.2008
(51) Int. Cl.: A61B 1/00, A61B 19/00

(54) **Method of rinsing endoscopes**
Verfahren zum Spülen von Endoskopen
Procédé de rinçage d'endoscopes

(30) Priority: 19.07.2007 JP 2007188560
(43) Date of publication of application: 21.01.2009
(73) Proprietor: FUJIFILM Corporation, Tokyo 106-0031 (JP)
(72) Inventor: Miyako, Kuniaki, Ashigara-kami-gun Kanagawa (JP)
(74) Representative: Klunker . Schmitt-Nilson . Hirsch

(56) References cited:
- WO-A-00/26334
- WO-A-01/56615
- US-A- 5 223 166
- US-A1- 2003 139 311
- US-A1- 2003 190 257
- US-A1- 2005 079 097

## Description

### BACKGROUND OF THE INVENTION

The present invention relates to a method of rinsing endoscopes after they have been cleaned; more particularly, it relates to a method of rinsing endoscopes in an automatic cleaner.

In the specification, simply "cleaning an endoscope" refers to a broad sense of "cleaning," i.e., a whole cleaning process of cleaning and disinfecting an endoscope including a step of cleaning an endoscope with detergent, a step of disinfecting the thus cleaned endoscope with disinfectant, and a step of rinsing the disinfected endoscope, while a "cleaning an endoscope with detergent" refers to a narrow sense of "cleaning," i.e., a specific cleaning step being only a step of cleaning an endoscope with detergent.

As is well known, the endoscope is inserted into cavities in the human body or other living bodies for use in various applications including the diagnosis and treatment of organs, and the sampling of specimens.

Endoscopes are used on more than one patient and repeatedly. Hence, used endoscopes require thorough hygienic management and to ensure complete prevention of troubles such as endoscope-mediated bacterial infection, they must be meticulously cleaned and disinfected after every use.

The endoscope cleaner has already been commercialized as an apparatus for automatic cleaning and disinfection of endoscopes (i.e. apparatus executing automatically a cleaning process of endoscopes) in a way that is not only accurate but also safe to the operator.

If the frequency of an endoscope's use is high, a plurality of endoscopes are used and then cleaned and disinfected sequentially. Hence, it is desirable that endoscopes can be cleaned and disinfected within the shortest possible time while maintaining high cleaning efficiency. The cleaning and disinfection (i.e. cleaning process) of endoscopes is generally accomplished by executing a step of cleaning with a detergent, a rinse step, a disinfection step, and another rinse step in the order written. A higher efficiency of the rinse steps contributes to shortening the time required to clean and disinfect endoscopes.

In the rinse step after the cleaning step, the dirt that has been removed in the cleaning step remains within the cleaning vessel and may potentially be re-deposited on the endoscope being cleaned. The re-deposited dirt will obviously prolong the rinse time. However, none of the conventional endoscope cleaners have been designed with special consideration being made about the re-deposition of dirt in the rinse step.

Now, JP 7-204591 A describes a cleaning system for removing oils and fats that are deposited on the surfaces of articles such as metal parts and non-metal parts, as well as for removing the flux used to solder on printed circuit boards; the system has three vessels, a cleaning vessel, a pre-rinse vessel, and a finishing rinse vessel. In this cleaning system, rinse effluent emerging from the pre-rinse vessel is subjected to ion-exchange so that any ionic substances contained in the rinse effluent are removed and that the halogen-free detergent and water contained in the rinse effluent are recycled for use as rinse water in the pre-rinse vessel. JP 7-204591 A also states that since the rinse water contains not only water but also the detergent component, the dirt components that cannot be thoroughly removed from the article of interest by rinse water that is solely composed of water can be efficiently removed and, at the same time, oils, fats and other dirt components are incorporated into micelles by the detergent component to assure high efficiency in preventing those dirt components from being re-deposited on the article of interest.

US 5,223,166 discloses a method of rinsing an endoscope comprising the following steps after completion of a cleaning step using a detergent which includes a surfactant: charging water into a vessel to fill said vessel with a first rinse water; rinsing said endoscope placed in said vessel with said first rinse water; supplying water into said vessel after rinsing with said first rinse water to fill said vessel with a second rinse water; and rinsing said endoscope with said second rinse water wherein when filling said vessel with said second rinse water, only water is supplied into said vessel so that said second rinse water contains no surfactant.

WO 01/56615 discloses a method for rinsing an endoscope wherein following a cleaning step, a first rinse step using a mixture of water and a surfactant is used. Subsequently, in a second rinse step, the rinse water contains no surfactant.

US 2005/0079097 discloses a method for rinsing an endoscope wherein a cleaning step involving a cleaning agent which contains surfactants is followed by two rinsing steps. The first rinsing step is performed with cold deionised water. The second rinsing step is performed with heated deionised water.

### SUMMARY OF THE INVENTION

However, according to the technique described in JP 7-204591 A, the detergent component carried into the pre-rinse vessel as a deposit on the article being cleaned is not removed but simply recycled for further use in the pre-rinse vessel, so the concentration of the detergent in the pre-rinse vessel is not held constant. Hence, satisfactory results cannot always be assured in preventing the dirt components from being re-deposited on the article of interest.

As a further problem, most of the conventional endoscope cleaners use a single vessel to perform cleaning, rinse and disinfection and each time the cleaning and disinfecting cycle ends, the processing fluid in the vessel is replaced. Hence, the endoscope cleaner does not cause the detergent component to be built up in the rinse step after the cleaning step and the concentration of the detergent component in the rinse water is always so low that it is not likely to be effective in preventing re-deposition of the dirt components.

An object, therefore, of the present invention is to solve the above-mentioned problems of the prior art by providing a method of rinsing endoscopes, in which the action of a surfactant contained in the detergent is effectively utilized in a rinsing treatment of the endoscope while the endoscope is rinsed to thereby ensure that re-deposition of dirt is suppressed to enhance the overall cleaning efficiency.

In order to attain the above-described object, the present invention provides a method of rinsing an endoscope in an endoscope cleaner placed in a vessel comprising:
charging a surfactant into a vessel in addition to water to fill the vessel with a first rinse water containing the surfactant;
rinsing the endoscope placed in the vessel with the first rinse water containing the surfactant;
supplying only water into the vessel after rinsing with the first rinse water to fill the vessel with a second rinse water that does not contain the surfactant; and
rinsing the endoscope with the second rinse water that does not contain the surfactant.

Preferably, the surfactant to be charged in addition to the water is adjusted to such an amount that a concentration of the surfactant in the first rinse water in the vessel is near a critical micelle concentration.

Preferably, water as supplied from a water supply is used at ordinary temperature in at least all steps of rinsing with the first rinse water whereas water as supplied from the water supply is used after being heated to an elevated temperature in part or all of steps of rinsing with the second rinse water.

And, preferably, overflow rinsing is first performed such that the endoscope is rinsed with rinse water that is partly drained from the vessel while the thus drained rinse water is compensated by a fresh supply of water, and, subsequently, pool rinsing is performed such that the endoscope is rinsed with a fresh water fully supplied in the vessel after the rinse water has been entirely drained from the vessel.

Here, preferably, the overflow rinsing is performed such that the endoscope is rinsed with the first rinse water in the vessel by draining partly the first rinse from the vessel while the thus drained first rinse water is compensated by the fresh supply of water, and the pool rinsing is performed such that the endoscope is rinsed with the second rinse water filled into the vessel by supplying the fresh water into the vessel after the first rinse water has been entirely drained from the vessel.

Preferably, the surfactant is charged into the vessel while the water is poured into the vessel, so that the vessel is filled with the first rinse water.

Preferably, the surfactant is charged continuously or intermittently into the vessel in accordance with supply of the water poured into the vessel.

Preferably, only the water is supplied into the vessel after the first rinse water has been entirely drained from the vessel, so that the vessel is filled with the second rinse water.

Preferably, the water is supplied from a water supply.

Preferably, the water is tap water supplied from a faucet.

Preferably, the surfactant is an anionic surfactant.

Preferably, the surfactant is a surfactant contained by a detergent used for cleaning of the endoscope.

And, preferably, a detergent used for cleaning of the endoscope uses for adjustment of the surfactant in the first rise water.

According to the present invention, the rinsing of an endoscope starts with adding a surfactant to water so that it is first rinsed with the water containing the surfactant, and then rinsed with water only. Because of this procedure, the action of the surfactant is effectively utilized so that the re-deposition of dirt is effectively suppressed to enhance the rinsing efficiency.

According to an embodiment of the present invention, the surfactant is added to water in such an amount that its concentration is close to the critical micelle concentration and this allows the surfactant in the rinse water to exhibit its action to the fullest extent so that the re-deposition of dirt is effectively suppressed to enhance the rinsing efficiency.

According to another embodiment of the present invention, the water in the surfactant-containing rinse water is used at ordinary temperature, namely, at the temperature at which it has been supplied from the faucet (water supply), whereas rinsing with water alone uses rinse water at a higher temperature; this provides effectiveness for suppressing the re-deposition of dirt in the first half of the rinse step whereas in the second half of the rinse step, it provides effectiveness for promoting the removal of the surfactant deposited on the endoscope, with the result that the rinse efficiency is again enhanced.

According to yet another embodiment of the present invention, the effects just described above are of course obtained and, what is more, overflow rinsing is performed in the first half of the rinse step but pool rinsing is performed in the second half of the rinse step, and this contributes to a further enhancement of the rinse efficiency.

Thus, according to the present invention, by enhancing the rinse efficiency in the respective embodiments described above, the overall cleaning efficiency can be enhanced.

### BRIEF DESCRIPTION OF THE DRAWINGS

FIG. 1 is a block diagram showing a schematic configuration of an exemplary endoscope cleaner for implementing the method of the present invention for rinsing endoscopes.
FIG. 2 is a perspective view of an endoscope.
FIG. 3 is a block diagram showing an outline of the pipelines in the endoscope cleaner shown in FIG. 1.
FIG. 4 is a block diagram showing in concept a schematic configuration of the control unit.
FIG. 5 is a flow sheet showing the method of the present invention for cleaning endoscopes.
FIG. 6 shows in concept how solid dirt is removed.
FIG. 7 shows in concept how oily dirt is removed.
FIG. 8 shows in concept how the article being cleaned and the dirt are repelled from each other by the electrostatic force.
FIG. 9 is a graph showing how the rinse time or the number of replacements of rinse water is related to the residual amount of a surfactant.

### DETAILED DESCRIPTION OF THE INVENTION

The endoscope rinsing method according to the present invention is described below in detail with reference to the preferred embodiments depicted in the accompanying drawings.

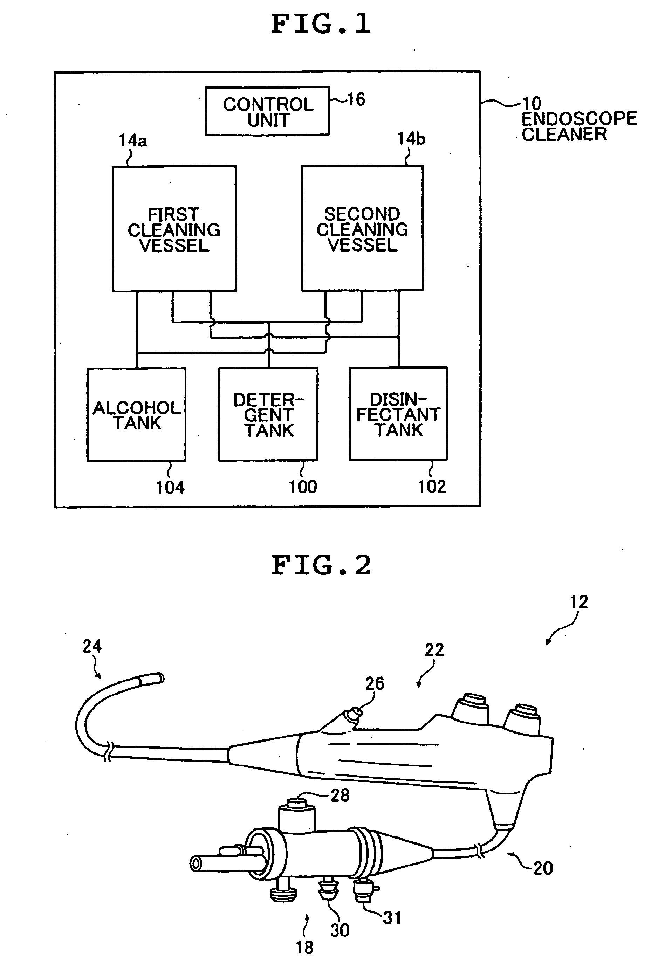

FIG. 1 is a block diagram showing a schematic configuration of an exemplary endoscope cleaner for implementing the method of the present invention for rinsing endoscopes. The endoscope cleaner which is generally indicated by 10 in FIG. 1 (and which is hereinafter referred to as cleaner) is such an apparatus that two units of an endoscope which is generally indicated by 12 in FIG. 2 can be individually cleaned and disinfected. As shown, cleaner 10 comprises a first cleaning vessel 14a, a second cleaning vessel 14b, a control unit 16, a detergent tank 100, a disinfectant tank 102, and an alcohol tank 104. In the embodiment under consideration, the cleaner 10 is equipped with two cleaning vessels but it may be equipped with only one cleaning vessel or even three or more cleaning vessels.

Endoscope 12 is of a conventionally known type and, as shown in FIG. 2, it comprises a connector section 18 to be connected to a light source, a universal cord section 20 connected to the connector section 18, a manipulating section 22 that is connected to the universal cord section 20 for adjusting the viewing angle and effecting various operations such as suction and air insufflation or water purge, and an insertion section 24 that is connected to the manipulating section 22 and is to be inserted into body cavities of a patient.

A light guide is accommodated within the endoscope 12 to extend from the end of the connector section 18 to the distal end of the insertion section 24; an air insufflation or water purge tube that forms an air insufflation or water purge channel extends from an air insufflation or water purge channel opening 28 in the connector section 18 to the distal end of the insertion section 24; and a suction tube that forms a suction channel extends from a suction channel opening 30 in the connector section 18 to the distal end of the insertion section 24. In addition, a forceps channel is provided to extend from a forceps channel opening 26 in the manipulating section 22 to the distal end of the insertion section 24. In some models, a forceps raising channel is provided to extend from the forceps channel opening 26 in the manipulating section 22 to the distal end of the insertion section 24. In addition, a pressurizing opening 31 for water leak detection is provided at the connector section 18.

The procedure of cleaning and disinfecting the endoscope 12 involves thorough cleaning and disinfection of not only its exterior but also the interiors of the forceps channel, air insufflation or water purge channel, suction channel, and the forceps raising channel. During this procedure, the electronic components for imaging need be fully protected from the cleaning water and the processing fluid.

Referring to cleaner 10 in FIG. 1, each of the first cleaning vessel 14a and the second cleaning vessel 14b is a vessel for cleaning and disinfecting one endoscope 12 and these vessels have the same configuration. The control unit 16 controls the steps in the cleaning and disinfecting procedure to be followed in the first cleaning vessel 14a and the second cleaning vessel 14b.

The detergent tank 100 is a part where a detergent for cleaning the endoscope is held. The detergent is used in the first cleaning vessel 14a and the second cleaning vessel 14b after it is diluted with water by a predetermined number of folds. The diluted detergent (processing fluid) is used to clean the endoscope 12 and discharged as an effluent after each cleaning step.

The disinfectant tank 102 is a part where the disinfectant is held. In the case where the disinfectant to be used in the cleaner 10 is of such a type that it can withstand more than one cycle of disinfection, the disinfectant supplied from the disinfectant tank 102 to the first cleaning vessel 14a or the second cleaning vessel 14b is recovered into the disinfectant tank 102 after disinfection. After it is used for a predetermined number of times, the disinfectant is discharged as an effluent.

The alcohol tank 104 is a part where an alcohol for alcohol flushing is held.

The cleaner 10 is equipped with one disinfectant tank 102 for the two cleaning vessels 14a and 14b, so these two cleaning vessels 14a and 14b share one and the same disinfectant tank 102. The cleaner 10 is also equipped with one unit each of detergent tank 100 and alcohol tank 104, which are shared by the two cleaning vessels 14a and 14b. In FIG. 1, the lines connecting each of the first cleaning vessel 14a and the second cleaning vessel 14b to the detergent tank 100, disinfectant tank 102 and the alcohol tank 104 indicate that these components are connected via pipes.

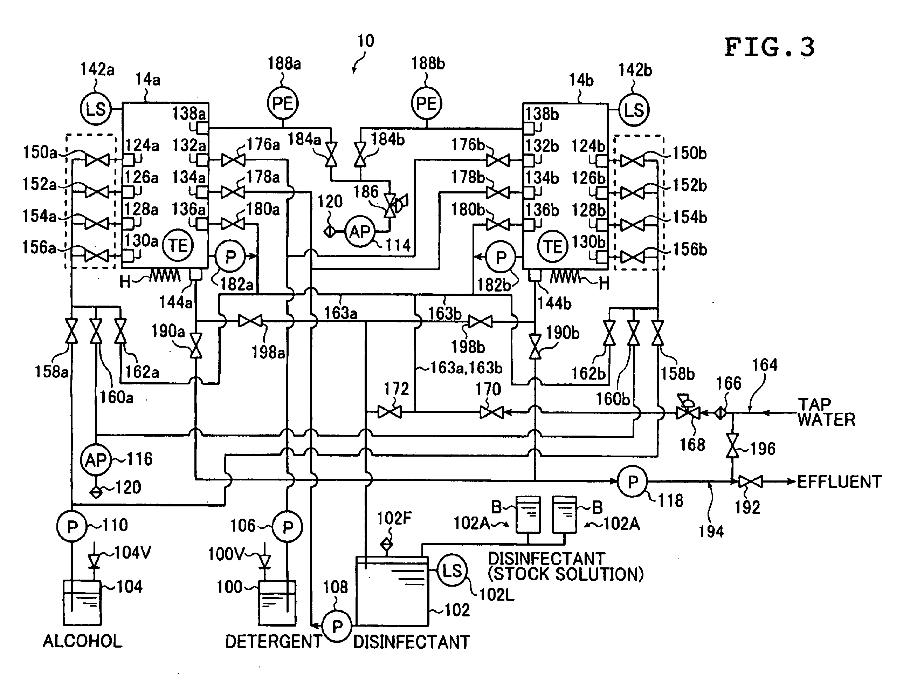

FIG. 3 is a block diagram showing an outline of the pipelines in the endoscope cleaner 10 shown in FIG. 1. As shown in FIG. 3, the cleaner 10 has only one unit each of a detergent pump 106, a disinfectant pump 108, and an alcohol pump 110; the detergent pump 106 supplies the detergent from the detergent tank 100 to the cleaning vessels 14a and 14b; the disinfectant pump 108 supplies the disinfectant from the disinfectant tank 102 to the cleaning vessels 14a and 14b; and the alcohol pump 110 supplies the alcohol from the alcohol tank 104 to the cleaning vessels 14a and 14b; each of the detergent pump 106, disinfectant pump 108, and the alcohol pump 110 is shared by the two cleaning vessels 14a and 14b.

These pumps may be of any various known types but it is of course preferred to use metering pumps. If the respective tanks are positioned below the cleaning vessels 14a and 14b, it is preferred to use self-priming metering pumps such as diaphragm pumps.

The cleaner 10 also has only one unit each of a first air pump 114, a second air pump 116, and a drain pump 118; the first air pump 114 is used to detect any water leak from the respective channels through the endoscope 12; the second air pump 116 is used to supply air into the respective channels through the endoscope 12; the drain pump 118 is used to drain the cleaning vessels 14a and 14b of water or the processing fluid; each of the first air pump 114, second air pump 116, and the drain pump 118 is shared by the two cleaning vessels 14a and 14b. Each of the first air pump 114 and the second air pump 116 is provided with an air filter 120 at the air intake.

In the illustrated case, the disinfectant tank 102 is provided with a level sensor 102L for measuring the volume of the disinfectant in the tank and an attachment 102A for mounting a bottle B that is filled with the disinfectant (stock solution) which is to be supplied into the disinfectant tank 102. In the illustrated exemplary case, the cleaner 10 is equipped with two attachments 102A, 102A. The disinfectant tank 102 is also equipped with a masking filter 102F that prevents the smell of the disinfectant from leaking to the outside. If desired, the disinfectant tank 102 may further have an air filter for preventing dust, miscellaneous germs and other foreign matter from mixing into the disinfectant tank 102.

The cleaner 10 may be so adapted that the disinfectant bottle B can be kept mounted until it is replaced by the next charge of disinfectant; in this case, the disinfectant bottle B acts as a lid for the attachment 102A, hence, for the disinfectant tank 102.

The detergent tank 100 is provided with a check valve 100V to prevent accidental draining of the detergent from the detergent tank 100, and the alcohol tank 104 is also provided with a check valve 104V to prevent accidental draining of the alcohol from the alcohol tank 104.

The first cleaning vessel 14a and the second cleaning vessel 14b have basically the same configuration and many parts of the piping systems are also the same; therefore, on the following pages, only the first cleaning vessel 14a will be described and as regards the second cleaning vessel 14b, the numerals or symbols for the corresponding constituents will be parenthesized and only those parts that have different configurations than the first cleaning vessel 14a will be explained.

Provided within the first cleaning vessel 14a (second cleaning vessel 14b) are a forceps port 126a (126b) for establishing connection to the forceps channel opening 26 in the endoscope 12, an air insufflation or water purge port 128a (128b) for establishing connection to the air insufflation or water purge channel opening 28 in the endoscope 12, and a suction port 130a (130b) for establishing connection to the suction channel opening 30 in the endoscope 12. In the case of an endoscope having a forceps raising channel, a forceps raising port 124a (124b) is also provided to establish connection to the forceps raising channel opening.

Also provided within the first cleaning vessel 14a (second cleaning vessel 14b) are a detergent inlet 132a (132b) for introducing the detergent, a disinfectant inlet 134a (134b) for introducing the disinfectant, and a water supply inlet 136a (136b) for introducing water from a water supply e.g. tap water from a city water system, as well as an air inlet 138a (138b) through which air is introduced for water leak detection, and a drain outlet 144a (144b).

The first cleaning vessel 14a (second cleaning vessel 14b) is also provided with a level sensor 142a (142b) for detecting the volume of the fluid within the vessel, a thermometer TE for measuring the temperature of the fluid in the vessel, and a heater H for heating the fluid in the vessel. The heater H may suffice to be capable of heating the fluid (processing fluid) within the first cleaning vessel 14a and may be any known heater that can be installed either within or outside the first cleaning vessel 14a.

The level sensor 142a (142b) may be exemplified by one that is capable of detecting the volume of the fluid at four levels; alternatively, four level sensors may be provided.

The forceps raising port 124a (124b) is connected via a valve 150a (150b) to valves 158a, 160a, and 162a (158b, 160b, and 160b); the forceps port 126a (126b) is connected to these valves via a valve 152a (152b); the air insufflation or water purge port 128a (128b) is connected to these valves via a valve 154a (154b); and the suction port 130a (130b) is connected to these valves via a valve 156a (156b).

Note that the valves to be used in the cleaner 10 are not limited in any particular way and any known valves that can be opened or closed automatically, such as electromagnetic valves or electrically operated valves, may be employed. It should, however, be mentioned that valves for draining the effluent from the cleaning vessels 14a and 14b or valves to be provided on lines (pipes) for returning the disinfectant to the disinfectant tank are preferably of an electrically operated type for such reasons as the smallness of the dead space within the valves.

The valve 158a (158b) is connected to an alcohol pump 110 associated with the alcohol tank 104.

The valve 160a (160b) is connected to the second air pump 116 for introducing air into the respective channels through the endoscope 12.

The valve 162a (162b) is connected to a water supply line 164 for supplying tap water to the relevant sites in the cleaner 10.

The water supply line 164 is connected to a drinking water faucet or the like of a city water system (water supply) for supplying tap water into the cleaner 10. As shown in FIG. 3, the water supply line 164 has, in order from the upstream end, a filter 166, a reducing valve 168, a first valve 170 and a second valve 172; the filter 166 serves to prevent contamination with foreign matter, and the reducing valve 168 serves to prevent undue pressure buildup in the piping system in the apparatus.

The pipe from the valve 162a (162b) is connected to somewhere between the first valve 170 and the second valve 172 on the water supply line 164. In the following description, this pipe which extends from the valve 162a (162b) to somewhere between the first valve 170 and the second valve 172 shall be referred to as a water supply pipe 163a (163b) for the sake of convenience. The water supply pipe 163a (163b) forms a branch in the middle of the way and this branch is connected to a circulating pump 182a (182b) to be described later that is associated with the first cleaning vessel 14a (second cleaning vessel 14b) and to a valve 180a (180b) also described later that is provided at the water supply inlet 136a (136b).

The second valve 172 is connected to the disinfectant tank 102 and to a valve 190a (190b) connected to the drain outlet 144a (144b) on the first cleaning vessel 14a (second cleaning vessel 14b).

The detergent inlet 132a (132b) is connected to the detergent pump 106 via a valve 176a (176b). The disinfectant inlet 134a (134b) is connected to the disinfectant pump 108 via a valve 178a (178b). In addition, the water supply inlet 136a (136b) is connected to the water supply pipe 163a (163b) via the valve 180a (180b). In other words, the branch pipe that diverges from the water supply pipe 163a (163b) is connected to the valve 180a (180b), hence, to the water supply inlet 136a (136b).

The first cleaning vessel 14a (second cleaning vessel 14b) has the circulating pump 182a (182b) connected thereto. By means of the circulating pump 182a (182b), the fluid in the first cleaning vessel 14a (second cleaning vessel 14b) is supplied to the branch pipe that diverges from the water supply pipe 163a (163b) to extend to the valve 180a (180b), hence, to the water supply inlet 136a (136b).

The air inlet 138a (138b) through which air is introduced for water leak detection is connected via a valve 184a (184b) to a reducing valve 186 connected to the first air pump 114.

A pressure gage 188a (188b) is provided on the pipe extending from the air inlet 138a (138b) to the valve 184a (184b). Note that the pressure gage 188a (188b) is preferably a pressure transmitter or the like that delivers a signal to the first air pump 114 at the point in time whe n a predetermined pressure is reached.

The drain outlet 144a (144b) is connected to the drain pump 118 via a valve 190a (190b).

The drain pump 118 forces the fluid or the like within the cleaning vessels 14a and 14b to be sent to a drain line 194 having a valve 192. The water supply line 164 and the drain line 194 are connected via a bypass valve 196 in such a way that a site upstream of the filter 166 on the water supply line 164 is connected to a site upstream of the drain line 194 on the drain line 94.

The pipe between the drain outlet 144a (144b) and the valve 190a (190b) forms a branch in the middle of the way, which is connected via a valve 198a (198b) to the second valve 172 on the water supply line 164 and to the disinfectant tank 102. The pipeline extending from the drain outlet 144a (144b) on the cleaning vessel 14a (14b) to the disinfectant tank 102 combines with the valve 198a (198b) to constitute a disinfectant recovery means in the present invention.

Described above is the general layout of the pipelines in the cleaner 10.

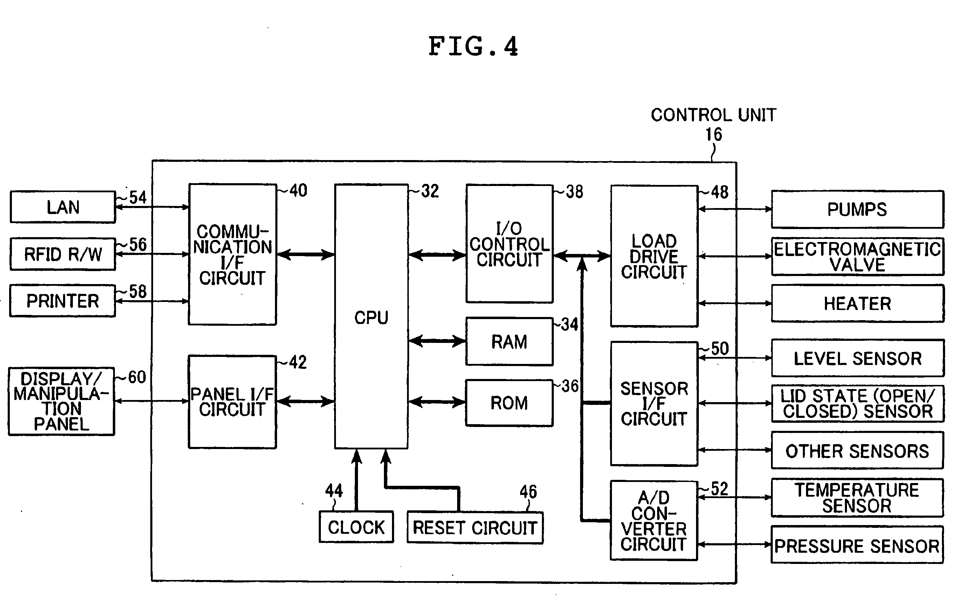

The control unit 16 controls the steps in the cleaning and disinfecting procedure to be performed in the first cleaning vessel 14a and the second cleaning vessel 14b. FIG. 4 is a block diagram showing in concept a schematic configuration of the control unit 16.

As shown in FIG. 4, the control unit 16 has a CPU 32, a RAM 234, a ROM 36, an I/O control circuit 38, a communication I/F circuit 40, a panel I/F circuit 42, a clock 44, a reset circuit 46, a load drive circuit 48, a sensor I/F circuit 50, and an A/D converter circuit 52.

The CPU 32 is for controlling the cleaning and disinfecting treatment as it is performed in the cleaner 10, and two cleaning vessels, the first cleaning vessel 14a and the second cleaning vessel 14b, are controlled by one CPU 32.

The ROM 36 stores a variety of application programs including a control program for controlling the cleaning and disinfecting treatment. The stored various application programs including the control program are read from the ROM 36 by means of the CPU 32 and set in the RAM 34. The RAM 34 stores data on the history of cleaning and disinfecting operations previously performed by the cleaner 10. If desired, the ROM 36 may be so adapted as to store only the control program for controlling the cleaning and disinfecting treatment that is associated with the number of the cleaning vessels the cleaner 10 has; in this case, the CPU 32 will always read that program from the ROM 36. Alternatively, the ROM 36 may store a plurality of control programs for controlling the cleaning and disinfecting treatment that are associated with various vessel arrangements ranging from the use of one cleaning vessel to the use of a given number of cleaning vessels; in this case, the CPU 32 will select the program that is associated with the actual vessel arrangement the cleaner 10 has and will read that program from the ROM 36.

Alternatively, variations of the control program for controlling the cleaning and disinfecting treatment may be stored in the ROM 36 such that in response to a command entered by the operator or following the choice it makes in association with the actual system configuration, the CPU 32 will choose the appropriate program and read it from the ROM 36.

The load drive circuit 48 drives the pumps (106, 108, 110, etc.), electromagnetic valves (150a, 152a, 154a, 156a, 198a, etc.) and the heater (H) that are shown in FIG. 3.

The sensor I/F circuit 50 is an interface for the level sensors (102L, 142a, 142b) that detect the fluid levels in the tanks and cleaning vessels, the sensors that detect the opening or closing of the lids on the cleaning vessels 14a and 14b, and any other sensors that are provided for the cleaner 10.

The A/D converter circuit 52 performs A/D conversion on the analog outputs from the temperature sensor (TE) and the pressure sensor (PE).

The communication I/F circuit 40 is a circuit for providing a communication interface with a LAN connector 54, a RFID R/W unit 56, and a printer 58 that are provided in the cleaner 10.

The cleaner 10 uses the LAN connector 54 to have the control unit 16 connected to, for example, a network in a hospital for two-way communication of the data on the history of cleaning and disinfecting operations previously performed by the cleaner 10.

The RFID R/W unit 56 uses a RFID (radio-frequency identification system) to read or write the information about cleaning and disinfecting operations. For instance, the RFID R/W unit 56 can access an IC tag on the endoscope 12 to read the data on the history of cleaning operations the cleaner 10 has performed; alternatively, after cleaning and disinfection have been performed by the cleaner 10, the RFID R/W unit 56 can write the data on that treatment into the IC tag on the endoscope 12. Furthermore, the RFID R/W unit 56 can access an IC tag containing the information that identifies the operator who is responsible for cleaning the endoscope 12 and read that information out of this IC tag; alternatively, the RFID R/W unit 56 can write the history of cleaning operations the operator has executed into the operator's IC tag. In response to a control command issued from the CPU 32, the various kinds of data that have been read by the RFID R/W unit 56 out of the IC tag can be stored in the RAM 34 as data on the history of cleaning operations or they can be sent to the network via the LAN connector 54.

The printer 48 can print history management data. The printer 48 may be built in the cleaner 10 or it may be an external printer.

The panel I/F circuit 42 is an interface with a display/manipulation panel 60 on the cleaner 10. The display/manipulation panel 60 displays information about the cleaner 10 and it also functions as a touch panel on which the operator can enter commands.

Each of the steps in the cleaning and disinfecting procedure (cleaning process) to be followed by the cleaner 10 is controlled by the control unit 16. The control unit 16 depends on the CPU 32 for reading a preset control program for controlling the cleaning and disinfecting treatment out of the ROM 36 and, in accordance with that control program, controls the pumps, valves, sensors and other components in the cleaner 10 to execute each of the steps in the cleaning and disinfecting procedure.

We next describe how the endoscope 12 is cleaned and disinfected by the cleaner 10. Again, the following description focuses on the first cleaning vessel 14a but the second cleaning vessel 14b can be operated in entirely the same way to clean and disinfect the endoscope. It should also be understood that in the following description of the treatment that is performed by each of the steps involved, all valves except those which are described as being "opened" remain closed and that all pumps except those which are described as being "driven" remain off.

In the cleaner 10, the endoscope 12 is cleaned and disinfected by the basic steps of cleaning with a detergent, rinse, disinfecting with a disinfectant, and rinse in the order written.

First, the operator (technician) sets up the endoscope 12 at a specified position in the first cleaning vessel 14a; the operator then connects the forceps channel opening 26 on the endoscope 12 to the forceps port 126a, the air insufflation or water purge channel opening 28 to the air insufflation or water purge port 128a, and the suction channel opening 30 to the suction port 130a; if the endoscope 12 has a forceps raising channel, the operator also connects the forceps raising channel opening to the forceps raising port 124a.

Note that these connecting operations may be performed by known means commonly practiced on endoscope cleaners, for example, by using connectors or the like.

When the setting up of the endoscope 12 is completed and a command for the start of a cleaning operation is entered, the cleaner 10 first performs the cleaning step.

To begin with, the reducing valve 168 and the first valve 170 on the water supply line 164 as well as the valve 180a connecting to the water supply inlet 136a are opened so that a predetermined amount of tap water that flows through the water supply line 164 and the water supply pipe 163a is introduced into the first cleaning vessel 14a via the water supply inlet 136a (the introduction of tap water).

When the predetermined amount of tap water has been introduced, the valve 176a connecting to the detergent inlet 132a is opened and the detergent pump 106 is driven so that a predetermined amount of the detergent is supplied from the detergent tank 100 through the detergent inlet 132a into the first cleaning vessel 14a (the introduction of the detergent).

If necessary, following the introduction of tap water in the cleaning step, namely, at some point in time between the introduction of tap water and the introduction of the detergent, the step of water leak detection (to be described later) may be performed.

If no step of water leak detection is carried out, the introduction of tap water and the introduction of the detergent may be performed in parallel.

When the predetermined amounts of tap water and detergent have been introduced into the first cleaning vessel 14a, the valve 162a is opened, the circulating pump 182a is driven, and in an exemplary case, the valve 150a connecting to the forceps raising port 124a (only when the forceps raising channel opening is connected to the forceps raising port 124a), the valve 152a connecting to the forceps port 126a, the valve 154a connecting to the air insufflation or water purge port 128a, and the valve 156a connecting to the suction port 130a are opened sequentially one by one for a predetermined period of time. The durations for which the valves are opened may be the same or different for the respective ports. The detergent forced into the endoscope 12 from the channel openings 26, 28, 30, etc. that are at one end of the respective channels flows through those channels and emerge from the distal end of the insertion portion 24 (see FIG. 2), which is at the other end of each channel, to return into the first cleaning vessel 14a.

In this way, the detergent in the first cleaning vessel 14a is circulated through the individual channels in the endoscope 12 to clean them successively (the cleaning of the channels).

When the cleaning of the channels ends, the valve 180a connecting to the water supply inlet 136a is opened and the circulating pump 182a is driven.

As a result, the detergent circulates around the endoscope 12 within the first cleaning vessel 14a to clean the exterior of the endoscope 12 (the cleaning of the exterior with the flowing fluid).

When cleaning of the exterior is performed for a predetermined period of time, the valves 190a and 192 are opened and the drain pump 118 is driven to drain the detergent from within the first cleaning vessel 14a (the draining of the detergent).

When the first cleaning vessel 14a is completely drained of the detergent, the valve 160a is additionally opened, with the valves 190a and 192 remaining open, and the second air pump 116 is driven and, what is more, the valve 150a connecting to the forceps raising port 124a, the valve 152a connecting to the forceps port 126a, the valve 154a connecting to the air insufflation or water purge port 128a, and the valve 156a connecting to the suction port 130a are opened sequentially one by one.

In this way, air is forced into the individual channels through the endoscope 12 via the forceps raising port 124a, forceps port 126a, air insufflation or water purge port 128a, and the suction port 130a, whereupon the endoscope 12 is emptied of any detergent that is left within the respective channels (air insufflation as part of the cleaning step).

The above-described cleaning step may be performed more than once. If the cleaning step is to be performed more than once, tap water and the detergent are again introduced into the first cleansing vessel 14a after the detergent is drained but before air insufflation is performed, so as to effect cleaning of the channels and cleaning of the exterior with the flowing fluid and the detergent is then drained. This procedure is repeated a predetermined number of times before air insufflation into each of the channels through the endoscope 12 is finally performed.

The foregoing procedure completes the cleaning step and, subsequently, rinse is performed as a post-cleaning step. The rinse step is the characterizing part of the present invention.

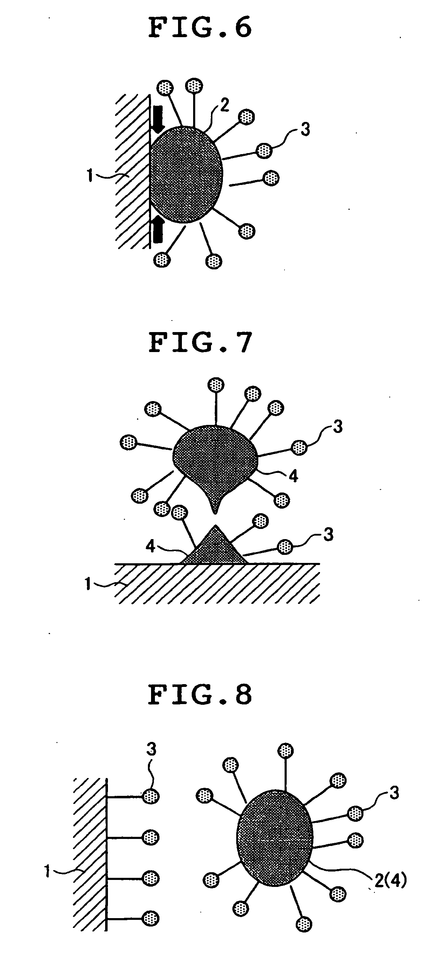

Before going into details of the rinse step, we describe the mechanism by which cleaning and disinfection are effected. FIG. 6 shows in concept how solid dirt is removed and FIG. 7 shows in concept how oily dirt is removed.

The case of solid dirt is first discussed. Suppose the detergent is applied in the above-described cleaning step where the article 1 which need be cleaned has a deposit of dirt 2. As FIG. 6 shows, the surfactant 3 in the detergent is adsorbed on the dirt 2 and the surface tension of water is lowered by the action of the surfactant, whereupon the moisture forces its way into the narrow space between the dirt 2 and the article 1, causing the dirt 2 to be easily separated from the article 1. Once the dirt 2 has separated from the article 1, the solids are retained within the aqueous solution by the dispersing action of the surfactant 3.

The case of oily dirt is next discussed. Suppose the detergent is applied in the above-described cleaning step where the article 1 which need be cleaned has a deposit of oily dirt 4; the surfactant 3 is adsorbed on the dirt 4 and the interfacial tension between the oil (oily dirt 4) and water is lowered but the interfacial tension between the oily dirt 4 and the article 1 does not change. Hence, as FIG. 7 shows, the interface between the oily dirt 4 and the article 1 decreases but the surface area of contact between the oily dirt 4 and the water is more likely to increase, causing the oily dirt 4 to be easily removed from the article 1. Once the oily dirt 4 has separated from the article 1, it is retained as the oil content within the aqueous solution by the emulsifying action of the surfactant 3. Note here that if the surfactant is present in a large amount, it also becomes possible to render the oil soluble.

As FIG. 8 shows, the surfactant 3 is also adsorbed on the article 1 which need be cleaned. Since the surfactant 3 is adsorbed on both the dirt 2 (or oily dirt 4) and the article 1, the article 1 and the dirt 2 repel each other to ensure that the dirt 2 that has separated from the article 1 will not be re-deposited on the article 1. Note that FIG. 8 refers to the case of an anionic surfactant and shows in concept how the article 1 and the dirt 2 are repelled from each other by the electrostatic force. A nonionic surfactant has the same action although it is less effective.

If the concentration of the surfactant within the water (aqueous solution) in the first cleaning vessel 14a exceeds a certain point, a micelle begins to form. This threshold concentration is called cmc, or critical micelle concentration. The cleaning power of the surfactant rises if its concentration is increased but beyond cmc, there is no further increase in its effectiveness. However, in the presence of dirt, the surfactant is adsorbed on the surface of the dirt and its concentration drops; hence, the general practice in the cleaning step is to allow for the drop in the concentration of the surfactant due to dirt and apply a more than necessary amount of the surfactant that is sufficient to make up for the anticipated drop in its concentration. Stated more specifically, the detergent is dosed in the cleaning step in such an amount that the concentration of the surfactant is in great excess of the critical micelle concentration (cmc), for example, more than twice the value of cmc.

It should also be noted that since oil becomes soluble in water only when it is incorporated into micelles, solubilization of the oil takes place at concentrations that are equal to or greater than cmc.

We now turn back to the procedure of cleaning and disinfecting the endoscope 12 in the cleaner 10. At the stage where the cleaning step has ended, not all of the dirt that had separated from the endoscope 12 (the article being cleaned) has been completely carried away by the effluent as the latter was drained out of the first cleaning vessel 14a but, instead, part of the dirt remains intact on the inner wall surfaces of the first cleaning vessel 14a or on the surface of the endoscope 12. In this instance, the dirt remaining in the interior of the first cleaning vessel 14a is kept covered with the surfactant and on account of the surfactant that also remains on the surface of the endoscope 12, the dirt that was separated in the cleaning step will not be immediately re-deposited on the endoscope 12.

However, the study of the present inventor showed that if the concentration of the surfactant were lowered abruptly when the dirt remained within the first cleaning vessel 14a at the initial stage of the rinse step following the cleaning step, re-deposition of the dirt on the endoscope 12 might be promoted.

Under the circumstances, the rinse method of the present invention is performed in such a way that the surfactant is applied at the initial stage of the rinse step to thereby ensure that the concentration of the surfactant is held within a specified range to suppress re-deposition of the dirt and that thereafter the surfactant is removed in the second half of the rinse step.

The dose of the surfactant to be applied in the rinse step is totally unlike the dose in the cleaning step and it is preferably adjusted to provide a concentration in the neighborhood of the critical micelle concentration. As mentioned above, the cleaning step takes into account the drop in the concentration of the surfactant due to dirt and the detergent is applied in an amount that can achieve a surfactant's concentration that is greater than the cmc by several factors; this is not the case for the rinse step that is effected after the cleaning step has already finished, and the concentration of the surfactant may suffice to be capable of suppressing re-deposition of the dirt, hence, it may be applied in amounts within a range that can maintain the critical micelle concentration.

For adjustment of the surfactant in the rinse step, the cleaner 10 uses the same detergent as is used in the cleaning step. The surfactant to be applied in the rinse step may be different from the detergent used in the cleaning step but use of the same detergent contributes to simplifying the system configuration and the maintenance of consumables.

The detergent for use in the cleaner 10 may occasionally contain not only the surfactant but also a builder, an enzyme, a bleaching agent, etc. The surfactant may be either anionic or nonionic, or both types of surfactants may be used. Enzymes include a protease, a carbohydrase and a lipase and part or all of these may be applicable; however, since the dirt in endoscopes is mainly composed of protein, it is generally the case for the detergent to contain a protease as the main enzyme component. If an alkali builder is to be contained for enhanced cleaning efficiency, a metal corrosion inhibitor is preferably incorporated in order to reduce any damage to the endoscope.

The concentration of the surfactant in the first cleaning vessel 14a is adjusted by the control unit 16.

To describe an example, the concentration of the surfactant for the case where tap water has been introduced to a predetermined level in the first cleaning vessel 14a after the start of the rinse step is preliminarily determined and the volume of the detergent to be supplied is preliminarily set on the basis of that information; the control unit 16 may then allow the preset volume of the detergent to be added to tap water.

Alternatively, an instrument for measuring the concentration of the surfactant is installed within the first cleaning vessel 14a and the control unit 16 looks to the value of measurement by the instrument to calculate the volume of the detergent to be supplied; the calculated volume of the detergent may then be added to tap water.

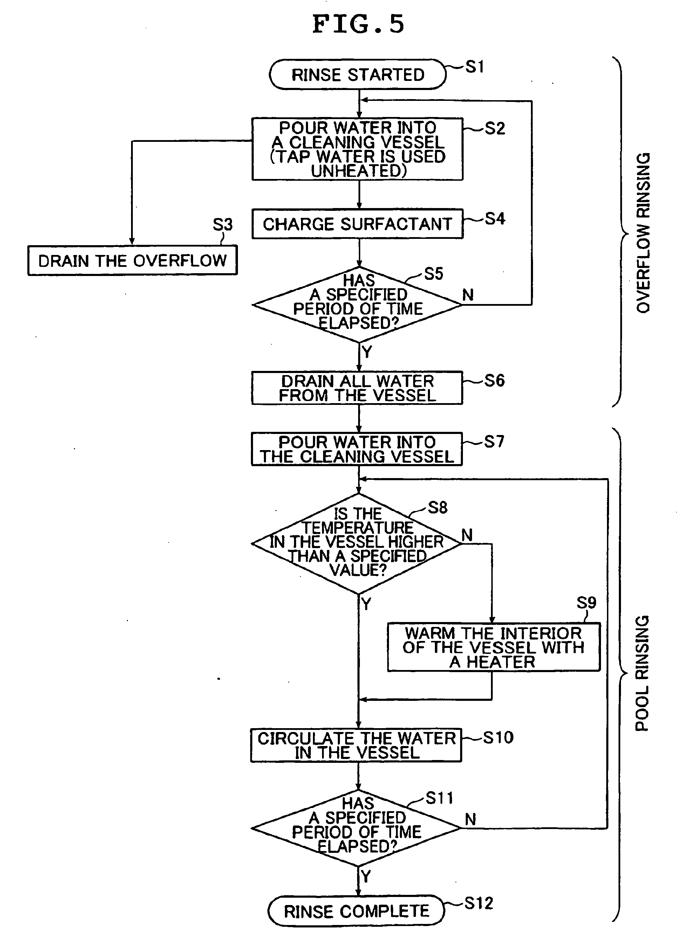

We next describe the rinse step after the cleaning step has ended in the cleaner 10. FIG. 5 is a flow sheet showing the respective sub-steps in supplying and draining water or liquid in the rinse step. To execute the rinse process or step, the control unit 16 controls the respective parts of the cleaner 16 to supply rinse water to both the interior and exterior of the endoscope 12 and, in addition, it follows the sequence of the sub-steps in FIG. 5 to control such factors as the replacement of rinse water in the first cleaning vessel 14a (14b) in the cleaner 10, the components of the rinse water, and the temperature of water.

As FIG. 5 shows, the rinse step after the cleaning step starts in sub-step S1 which is followed by sub-tep S2, where water is poured into the first cleaning vessel 14a. The control unit 16 opens the reducing valve 168, the first valve 170 and the valve 180a so that a predetermined amount of tap water is introduced into the first cleaning vessel 14a.

In a preferred embodiment of the present invention, water as supplied from the faucet is used in sub-step S2 at ordinary temperature without being heated. Tap water is preferably used without being heated because use of water with elevated temperature increases the likelihood that the dirt that has been removed in the cleaning step is re-deposited on the endoscope 12.

Generally speaking, the higher the temperature of water, the more effectively the dirt is removed but, on the other hand, the greater the chance of it being re-deposited. This may be explained as follows: the higher the temperature, the greater the molecular motion that occurs in the surfactant, making it difficult for the surfactant to form micelles or adhere to the article being cleaned, which results in a greater chance for the surfactant to be detached from the surface of the dirt or the article being cleaned.

This problem bears particular importance for the cleaner 10 which has a surfactant added in sub-step S4 (to be described later) in order to suppress the re-deposition of the dirt. If water with elevated temperature is supplied in sub-step S2, the water solubility of the surfactant, especially if it is nonionic, is so much lowered that it fails to exhibit its effect; hence, at least for the period of rinsing with water that contains the surfactant, it is preferred to use water with low temperature such as ordinary temperature. The suitable temperature range varies with the type of surfactant to be used and in the case of a nonionic surfactant, it is desirable to use a temperature not exceeding a value that is 15 to 20 °C higher than the cloud point.

In a certain case, the temperature of water may be elevated in the cleaning step in order to enhance the cleaning efficiency; in this instance, lowering the temperature of water in the rinse step is effective for suppressing the re-deposition of dirt.

When the water that began to be poured in sub-step S2 has reached a predetermined level in the first cleaning vessel 14a, the overflow is drained to the outside of the first cleaning vessel 14a in sub-step S3 but the pouring is continued. Specifically, an overflow outlet (not shown) communicating with the drain outlet 144a or the drain line 194 may be provided in a position corresponding to the predetermined water level in the first cleaning vessel 14a such that the amount of water that exceeds the predetermined level is discharged through that overflow outlet. In this case, a shutter or valve is preferably provided to disconnect the overflow outlet on the first cleaning vessel 14a from the drain line 194 such that the overflow outlet on the first cleaning vessel 14a is closed except in the case of draining the overflowing water. Alternatively, an overflow outlet through which the overflowing water is discharged and an electromagnetic valve for opening or closing it may be provided in a suitable position below the predetermined water level in the first cleaning vessel 14a; in this case, the opening or closing of the electromagnetic valve is controlled to control the draining of the overflowing water.

Since the pouring of water is continued in sub-step S2, the drained overflowing water is compensated by the freshly supplied water. In the way described above, overflow rinsing is effected through the loop of sub-step S2 to sub-step S5 including sub-step S3.

In sub-step S4, while the pouring of water into the first cleaning vessel 14a that started in sub-step S2 is continued, the surfactant begins to the charged into the first cleaning vessel 14a. The amount of the surfactant to be charged is adjusted in such a way that the concentration of the surfactant in the first cleaning vessel 14a is near the critical micelle concentration. The surfactant may be charged after water has been poured to reach the predetermined water level in the first cleaning vessel 14a; however, in order to ensure that the concentration of the surfactant in the first cleaning vessel 14a will not drop considerably, the surfactant preferably begins to be charged while water is also poured into the first cleaning vessel 14a. In addition, if overflow rinsing is effected as in the embodiment under consideration, the supply of the surfactant is preferably continuous or intermittent in order to control the concentration of the surfactant to lie within a predetermined range.

After the first cleaning vessel 14a is filled with the rinse water, i.e., tap water containing the surfactant (which is hereinafter sometimes referred to as the first rinse water), the rinse water is circulated not only into the respective channels through the endoscope 12 but also to its exterior as in the aforementioned cleaning step in order to rinse the endoscope 12.

First, the valve 162a is opened, the circulating pump 182a is driven, and the valves 150a, 152a, 154a and 156a are opened sequentially one by one so that the rinse water in the first cleaning vessel 14a is successively fed into the respective channels, whereupon as in the cleaning of the channels, rinse of the channels is performed by rinsing the respective channels through the endoscope 12. Thereafter, the valve 180a is opened and the circulating pump 182a is driven so that the rinse water is circulated in the first cleaning vessel 14a, whereupon as in the cleaning of the exterior with the flowing fluid, rinse of the exterior with the flowing fluid is performed by rinsing the exterior of the endoscope 12.

In the next sub-step S5, a question is asked whether a predetermined period of time has elapsed. This period of time is the time that has been set to allow for the overflow rinsing to be performed and it may be counted by the time elapsed since water began to be poured into the first cleaning vessel 14a in sub-step S2 or by the time elapsed since the predetermined water level in the first cleaning vessel 14a was reached in sub-step S2 and subsequent sub-steps. This period of time may be substantially equal to or greater than the time it takes for the above-described rinsing of the channels through the endoscope and rinsing of its exterior with the flowing fluid to be completed.

If the answer to the question asked in sub-step S5 is negative (the predetermined period of time has not elapsed), a decision for "no" is made in sub-step S5 and the process returns to sub-step S2, where the pouring of water for the overflow rinsing is continued. If the predetermined period of time has elapsed and a decision for "yes" is made in sub-step S5, the process goes to sub-step S6 where all water in the first cleaning vessel 14a is drained off. The control unit 16 opens the valves 190a and 192 and drives the drain pump 118 to effect draining in the rinse step as in the draining after cleaning.

When the draining ends, the process goes to sub-step S7 where water is again poured into the first cleaning vessel 14a. This time, the surfactant is not charged but tap water alone is used as rinse water (the second rinse water).

When the first cleaning vessel 14a is filled with the tap water (the second rinse water) up to the predetermined water level, the process goes to sub-step S8 where a check is made to see whether the temperature in the first cleaning vessel 14a is equal to or higher than a predetermined value. To be more specific, the control unit 16 receives a value detected with the thermometer TE provided within the first cleaning vessel 14a and compares it with the preset temperature value. If the temperature in the first cleaning vessel 14a is less than the predetermined value, a decision for "no" is made in sub-step S8 and the process goes to sub-step S9 where the interior of the first cleaning vessel 14a is heated with the heater H which is also provided within the first cleaning vessel 14a.

The temperature setting which serves as the reference for making a decision in sub-step S8 may be the temperature which is effective for causing the detergent, namely, the surfactant in the detergent to be removed from the endoscope 12 which is the article being cleaned. Generally speaking, the higher the temperature, the better but, on the other hand, damage might be caused to the endoscope; hence, the temperature setting needs to be not more than 60 °C.

Immediately after tap water began to be introduced in sub-step S7, a decision for "no" is made in sub-step S8 and heating is started in sub-step S9. And by the time the final stage of the rinse step is reached, the rinse water acquires the predetermined temperature. Thus, by raising the temperature of the rinse water in the last step of the rinse process, deposition of the detergent on the article being cleaned can be inhibited to achieve effective removal of the detergent.

When heating gets started in sub-step S9, the process goes to sub-step S10 with the heating operation being continued. If the temperature in the first cleaning vessel 14a is found to be equal to or higher than the predetermined value in sub-step S8, a decision for "yes" is made in sub-step S8 and the interior of the first cleaning vessel 14a is not heated but the process goes to sub-step S10.

In sub-step S10, the endoscope 12 is rinsed as in the rinse with the aforementioned first rinse water by circulating the second rinse water in the first cleaning vessel 14a to flow not only into the respective channels through the endoscope 12 but also to its exterior.

If desired, sub-steps S8 and S9 may be executed before the water that began to be poured in sub-step S7 reaches the predetermined water level; in this alternative case, tap water with ordinary temperature may begin to be warmed as it is poured into the first cleaning vessel 14a. It should, however, be noted that circulation of the rinse water in sub-step S10 is to be started after it has built up to the predetermined water level in the first cleaning vessel 14a.

In the next sub-step S11, a question is asked whether a predetermined period of time has elapsed. This period of time is the time that has been set to allow for pool rinsing to be performed and it may be counted by the time elapsed since the circulation of the rinse water started in sub-step S10. This period of time may be substantially equal to or greater than the time it takes for the rinsing of the channels through the endoscope and rinsing of its exterior with the flowing fluid to be completed in sub-step S10.

If the answer to the question asked in sub-step S11 is negative (the predetermined period of time has not elapsed), a decision for "no" is made in sub-step S11 and the process returns to sub-step S8. In sub-step S8, the temperature in the first cleaning vessel 14a is checked and if it has reached the predetermined value, the process does not go to sub-step S9 but heating with the heater H ends. If the temperature in the first cleaning vessel 14a is yet to reach the predetermined value, the process again goes to sub-step S9 and heating with the heater H continues.

This is how pool rinsing is performed through the loop of sub-steps S8 to S11 using the rinse water with which the first cleaning vessel 14a has been filled in sub-step S7.

If the answer to the question asked in sub-step S11 is affirmative (the predetermined period of time has elapsed), a decision for "yes" is made in sub-step S12 and the rinse step ends.

When the rinse step as a post-cleaning step ends, the valve 160a is opened, the second air pump 116 is driven, and the valves 150a, 152a, 154a and 156a are opened sequentially one by one so that air insufflation as part of the rinse step is performed in the same manner as air insufflation following the cleaning step.

Thus, in the rinse step depicted in FIG. 5, overflow rinsing is first performed, which is then followed by pool rinsing and this is a preferred embodiment of the present invention.

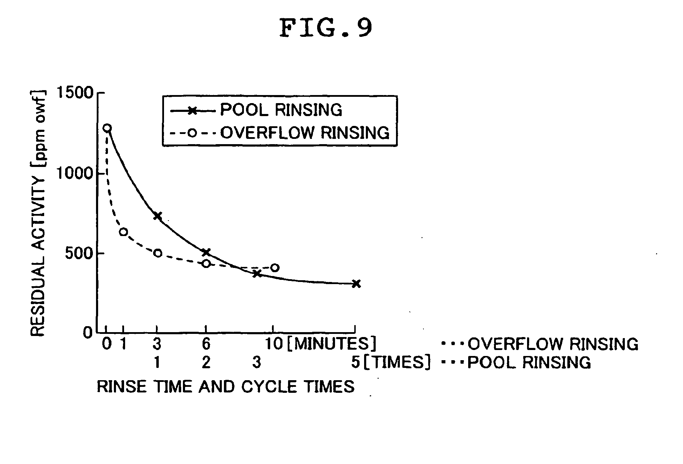

Generally speaking, overflow rinsing provides a higher cleaning efficiency within a short time in the initial period but, on the other hand, the final degree of cleaning that can be ultimately reached is higher in pool rinsing. FIG. 9 is a graph which, referring to the cleaning of a textile [Kondo et al., Shouhikagaku Gakkaishi (Journal of the Japan Research Association for Textile End-Uses, 12,257, 1971)], shows how the period of overflow rinsing or the number of replacements of rinse water in pool rinsing is related to the residual amount of a surfactant. The plausible explanation of the result shown in FIG. 9 would be as follows: when the concentration of dirt in the rinse water is high, it can be reduced more quickly by overflow rinsing but as it becomes lower, the ultimate concentration of dirt that can be reached by pool rinsing which involves complete replacement of the rinse water is lower than the value attained by overflow rinsing.

Under the circumstances, overflow rinsing is carried out at the initial rinse stage but pool rinsing is performed in the latter half of the rinse step and this enables a high cleaning efficiency to be attained in a short period of time.

In the cleaner 10, the rinse step is divided into two stages and the characteristics of the rinse water are controlled in terms of three factors, the use of the surfactant in the rinse water, the temperature of the rinse water, and the method of rinse water replacement. By thusly controlling the characteristics of the rinse water in both the first and second parts of the rinse step, re-deposition of dirt can be suppressed.

In the case shown above, the first half of the rinse step which consists of sub-steps S2 to S5 is allowed to differ from its second half which consists of sub-steps S7 to S11 in all of the three factors, the use of the surfactant in the rinse water, the temperature of the rinse water, and the method of the rinse water replacement.

However, the present invention is in no way limited to that particular case and control may only be performed to determine whether the surfactant should or should not be used in the rinse water, or alternatively, either the temperature of the rinse water or the method of rinse water replacement may only be controlled. In other words, the rinse step may be performed in a mode where the rinse water is not warmed or in a mode where either overflow rinsing or pool rinsing is performed throughout the rinse step.

If overflow rinsing is to be performed throughout the rinse process, sub-steps S6 and S7 may be omitted and in this case the following procedure may be taken: after the supply of the surfactant in sub-step S4 is ceased, the overflowing rinse water that contains the added surfactant is drained until the first cleaning vessel 14a contains only the tap water, whereupon the second half of the rinse step is started. If, on the other hand, pool rinsing is to be performed in all sub-steps of the rinse step, more than one cycle of pool rinsing may be carried out with one or more replacements of the rinse water in the first cleaning vessel 14a.

Making control as to whether the surfactant should or should not be used in the rinse water contributes to suppressing re-deposition of dirt on the endoscope 12 at the initial stage of the rinse step which involves a large amount of dirt.

In addition to this control over the use of the surfactant, the temperature of the rinse water may also be controlled to provide the following effects: low-temperature water is effective in reducing the amount of the surfactant that need be used at the initial stage of the rinse step whereas high-temperature water contributes to promoting the removal of the surfactant in the second half of the rinse step.

In addition to the control over the use of the surfactant, the method of rinse water replacement may also be controlled to facilitate the removal of the surfactant after it has been used at the initial stage of the rinse step.

In the example described above, all of these effects can be obtained.

When at least two of the three factors mentioned above are to be controlled, they may be controlled on different timings. For example, the temperature of the rinse water may be raised after rinsing with the surfactant-free rinse water (the second rinse water) is started and toward the end of the rinse step. If desired, overflow rinsing may be shifted to pool rinsing halfway through the process of rinsing with the surfactant-containing rinse water (the first rinse water) or, alternatively, overflow rinsing may be shifted to pool rinsing halfway through the process of rinsing with the surfactant-free rinse water (the second rinse water).

However, as already mentioned before, the surfactant fails to exhibit its effect if the temperature of the first rinse water is elevated; hence, it is preferably not the surfactant-containing rinse water (the first rinse water) but the surfactant-free rinse water (the second rinse water) that is to be warmed.

It should also be noted that since raising the temperature of the rinse water to a higher level during overflow rinsing is inefficient, pool rinsing is preferably performed if the rinse water is to be warmed.

The foregoing procedure completes the rinse as a post-cleaning step and, subsequently, the disinfecting step is performed.

In the disinfecting step, the valve 178a connecting to the disinfectant inlet 134a is first opened and the disinfectant pump 108 is driven to introduce a predetermined amount of the disinfectant into the first cleaning vessel 14a (the introduction of the disinfectant).

When the predetermined amount of the disinfectant has been introduced into the first cleaning vessel 14a, the interiors of the respective channels through the endoscope 12 are disinfected as in the above-described cleaning of the channels.

To state specifically, the valve 162a is opened and the circulating pump 182a is driven and, what is more, the valves 150a, 152a, 154a and 156a that are connected to the ports connecting to the respective channels through the endoscope 12 are opened sequentially one by one for a specified period of time.

As a result, the disinfectant in the first cleaning vessel 14a is circulated through the individual channels in the endoscope 12 to disinfect those channels with the disinfectant (the disinfection of the channels).

When the disinfection of the channels ends, the exterior of the endoscope 12 is disinfected as in the above-described cleaning of the exterior with the flowing fluid.

To state specifically, the valve 180a connecting to the water supply inlet 136a is opened and the circulating pump 182a is driven so that the disinfectant in the first cleaning vessel 14a is circulated around the endoscope 12 to disinfect its exterior with the disinfectant (the disinfection of the exterior with the flowing fluid).

Note here that if the disinfectant can be allowed to get into every part of the interior and the exterior of the endoscope 12 without circulating it, the disinfection of the channels and the disinfection of the exterior can be effected by immersing the endoscope 12 in the disinfectant for a specified period of time after it has been allowed to get to the necessary areas.

When the disinfection of the exterior with the flowing fluid has been conducted for a specified period of time, the valve 198a connecting to the water drawin outlet 144a is opened so that the disinfectant is returned into the disinfectant tank 102 (the recovery of the disinfectant).

In the illustrated case of the cleaner 10, a pump or the like is not used to recover the disinfectant but it is dropped under its own weight to return into the disinfectant tank 102.

When the disinfectant in the first cleaning vessel 14a has been recovered into the disinfectant tank 102, air is insufflated into the respective channels through the endoscope 12 as in the above-described air insufflation as part of the cleaning step.

To state specifically, the valve 160a is opened and the second air pump 116 is driven and, what is more, the valves 150a, 152a, 154a and 156a are opened sequentially one by one. In this way, air is forced into the individual channels through the endoscope 12 via the forceps raising port 124a, forceps port 126a, air insufflation or water purge port 128a, and the suction port 130a, whereupon the endoscope 12 is emptied of any disinfectant that is left within the respective channels (air insufflation as part of the disinfecting step).

The foregoing procedure completes the disinfecting step and, subsequently, rinse is performed as a post-disinfecting step.

The rinse as a post-disinfecting step is performed in basically the same way as the above-described rinse as a post-cleaning step.

To begin with, the reducing valve 168, the first valve 170 and the valve 180a are opened so that a predetermined amount of tap water is introduced into the first cleaning vessel 14a (the introduction of tap water).

When the introduction of tap water ends, the valve 162a is opened, the circulating pump 182a is driven, and the valves 150a, 152a, 154a and 156a are opened sequentially one by one for a specified period of time so that rinse of the channels is performed by rinsing the respective channels through the endoscope 12 with the tap water. Subsequently, the valve 180a is opened and the circulating pump 182a is driven so that rinse of the exterior with the flowing fluid is performed by rinsing the exterior of the endoscope 12 with the tap water.

When rinse of the exterior with the flowing fluid ends, the valves 190a and 192 are opened and the drain pump 118 is driven to effect draining as part of the rinse step. Thereafter, the valve 160a is opened, the second air pump 116 is driven, and the valves 150a, 152a, 154a and 156a are opened sequentially one by one so that air insufflation as part of the rinse step is performed to complete the rinse as a post-disinfecting step.

The above-described rinse step may be performed more than once. If the rinse step is to be performed more than once, tap water is again introduced into the first cleansing vessel 14a after the rinse water is drained but before air insufflation is performed, so as to effect rinse of the channels and rinse of the exterior with the flowing fluid. This procedure is repeated a predetermined number of times before air insufflation is finally performed.

Ending of the rinse step after the disinfecting step completes the cleaning of the endoscope 12 with the cleaner 10 and a visual display, an audible alarm or the like may be used to inform the operator that the cleaning of the endoscope 12 has ended.

As already mentioned, the cleaner 10 has the tanks, pumps and many other parts shared by the first cleaning vessel 14a and the second cleaning vessel 14b. However, except for the supply systems for the detergent and the like, the water supply line 164 and the drain line 194, the two cleaning vessels have mutually independent pipelines, so they can be operated to perform the same treatment at the same time or perform different treatments at the same time (the two vessels perform asynchronous treatments).

While the basic procedure of cleaning the endoscope 12 with the cleaner 10 has been described above, the cleaner 10 is capable of performing various treatments other than the above-described cleaning process.

For example, alcohol flushing may optionally be performed to ensure that the cleaned interiors of the respective channels through the endoscope 12 are dried at an accelerated speed.

Alcohol flushing is performed in the following manner; after the cleaning step ends, the valve 158a is opened and the alcohol pump 110 is driven, and what is more, the valve 150a connecting to the forceps raising port 124a, the valve 152a connecting to the forceps port 126a, the valve 154a connecting to the air insufflation or water purge port 128a, and the valve 156a connecting to the suction port 130a are opened sequentially one by one for a predetermined period of time.

Subsequently, as in the air insufflation conducted in each of the steps described above, the valve 160a is opened and the second air pump 116 is driven and, what is more, the valves 150a, 152a, 154a and 156a are opened sequentially one by one so that air is insufflated into the respective channels through the endoscope 12 to purge them of the alcohol and dry their interiors.

In addition, the drain outlet 144a and the valves 190a and 192 are opened and the drain pump 118 is driven to purge the first cleaning vessel 14a of the alcohol that has been drained into that vessel.

If another embodiment, the cleaner 10 may be so operated as to perform self-disinfection in which the water supply line 164, the drain line 194 and the like are disinfected with the disinfectant.

In this self-disinfecting step, the valve 178a connecting to the disinfectant inlet 134a is first opened and the disinfectant pump 108 is driven to introduce a predetermined amount of the disinfectant into the first cleaning vessel 14a.

Subsequently, the valve 190a connecting to the drain outlet 144a, the bypass valve 196, the reducing valve 168, the first valve 170, and the valve 180a connecting to the water supply inlet 136a are opened and the drain pump 118 is driven to circulate the disinfectant through the path including the water supply line 164 and the drain line 194.

In the illustrated case of the cleaner 10, a preferred embodiment is such that when the process of self-disinfection ends, the cleaner 10 is drained of the disinfectant and the disinfectant tank 102 is refilled with a fresh disinfectant.

To state specifically, when the disinfectant has been circulated for a specified period of time through the aforementioned path including the water supply line 164 and the drain line 194, the valves 190a and 192 are opened and the drain pump 118 is driven to drain the disinfectant. In addition, the valve 178a is opened and the disinfectant pump 108 is driven so that any disinfectant that might stay within the disinfectant tank 102 is drained off by being transferred into the first cleaning vessel 14a.

When all of the disinfectant in the cleaner 10 has been drained, the reducing valve 168, the first valve 170 and the second valve 172 are opened and a predetermined amount of tap water is charged into the disinfectant tank 102. Subsequently, the operator installs a disinfectant bottle B on each of the two attachments 102A. The disinfectant is introduced, typically under its own weight, into the disinfectant tank 102 so that it is refilled with the fresh disinfectant.

As already mentioned, after the introduction of tap water in the cleaning step, the cleaner 10 may optionally be so operated as to perform detection of any water leakage from the individual channels through the endoscope 12.

If the step of water leak detection is to be carried out, the endoscope 12 to be cleaned is set up in the first cleaning vessel 14a and at the same time the air inlet 138a (138b) is connected to the pressurizing opening 31 for leak detection that is provided on the endoscope 12 (see FIG. 2). When the introduction of tap water in the cleaning step ends, the first air pump 114 is driven and the reducing valve 186 as well as the valve 184a are opened. At the point in time when the pressure gage 188a reads a predetermined pressure, the drive of the first air pump 114 is stopped. This is preferably done automatically in response to a signal the pressure gage 188a delivers to the first air pump 114 in accordance with the result of pressure measurement.

When the pressurization ends, the endoscope 12 is visually checked for any air bubbles that might come out from it; if any air bubbles are seen coming out, any one of the channels through the endoscope 12 might have a leak, so the cleaning of the endoscope 12 is suspended at that point of time. Alternatively, if the pressure measured with the pressure gage 188a drops below a specified level within a specified period of time, any one of the channels through the endoscope 12 might again have a leak, so the cleaning of the endoscope 12 is suspended at that point of time. The pressure gage 188a may be so adapted that at the time when the pressure it measures drops below a specified level, it issues a warning to the effect that any one of the channels through the endoscope 12 has a leak.

While the method of the present invention for rinsing endoscopes has been described in detail on the foregoing pages, the present invention is by no means limited to the embodiments described above and it should be understood that various improvements and alterations can be made without departing from the scope and spirit of the present invention.

## Claims

1. A method of rinsing an endoscope placed in a vessel included in an endoscope cleaner (10) comprising the following steps after completion of a cleaning step using a detergent which includes a surfactant:
charging water into a vessel (14a, 14b) to fill said vessel (14a, 14b) with a first rinse water;
rinsing said endoscope placed in said vessel with said first rinse water;
supplying water into said vessel after rinsing with said first rinse water to fill said vessel with a second rinse water; and
rinsing said endoscope with said second rinse water wherein when filling said vessel with said second rinse water, only water is supplied into said vessel so that said second rinse water contains no surfactant, **CHARACTERIZED IN THAT**
when filling said vessel with said first rinse water, a surfactant is charged into said vessel in addition to the water so as to be adjusted to such an amount that a concentration of the surfactant in said first rinse water in said vessel is lower than a concentration of the surfactant in said detergent used in said cleaning step and is in the neighborhood of a critical micelle concentration.

2. The endoscope rinsing method according to claim 1 wherein water as supplied from a water supply is used at ordinary temperature in at least all steps of rinsing with said first rinse water whereas water as supplied from said water supply is used after being heated to an elevated temperature in part or all of steps of rinsing with said second rinse water.

3. The endoscope rinsing method according to claim 1 or 2, wherein
overflow rinsing is first performed such that said endoscope is rinsed with rinse water that is partly drained from said vessel while the thus drained rinse water is compensated by a fresh supply of water, and,
subsequently, pool rinsing is performed such that said endoscope is rinsed with a fresh water fully supplied in said vessel after said rinse water has been entirely drained from said vessel.

4. The endoscope rinsing method according to claim 3,
wherein
said overflow rinsing is performed such that said endoscope is rinsed with said first rinse water in said vessel by draining partly said first rinse water from said vessel while the thus drained first rinse water is compensated by said fresh supply of water, and
said pool rinsing is performed such that said endoscope is rinsed with said second rinse water filled into said vessel by supplying said fresh water into said vessel after said first rinse water has been entirely drained from said vessel.

5. The endoscope rinsing method according to any one of claims 1 to 4, wherein said surfactant is charged into said vessel while said water is poured into said vessel, so that said vessel is filled with said first rinse water.

6. The endoscope rinsing method according to claim 5, wherein said surfactant is charged continuously or intermittently into said vessel in accordance with supply of said water poured into said vessel.

7. The endoscope rinsing method according to any one of claims 1 to 6, wherein only said water is supplied into said vessel after said first rinse water has been entirely drained from said vessel, so that said vessel is filled with said second rinse water.

8. The endoscope rinsing method according to any one of claims 1 to 7, wherein said water is supplied from a water supply.

9. The endoscope rinsing method according to any one of claims 1 to 8, wherein said water is tap water supplied from a faucet.

10. The endoscope rinsing method according to any one of claims 1 to 9, wherein said surfactant is an anionic surfactant.

11. The endoscope rinsing method according to any one of claims 1 to 10, wherein said surfactant is a surfactant contained by a detergent used for cleaning of said endoscope.

12. The endoscope rinsing method according to any one of claims 1 to 11, wherein a detergent used for cleaning of said endoscope is used for adjustment of said surfactant in said first rise water.

## Patentansprüche

1. Verfahren zum Spülen eines Endoskops, welches in einem Gefäß platziert ist, das in einem Endoskopreiniger (10) enthalten ist, umfassend die folgenden Schritte nach Beendigung eines Reinigungsschritts unter Verwendung eines ein Tensid enthaltenden Reinigungsmittels:
Einfüllen von Wasser in ein Gefäß (14a, 14b), um das Gefäß (14a, 14b) mit einem ersten Spülwasser zu füllen;
Spülen des in dem Gefäß platzierten Endoskops mit dem ersten Spülwasser;
Zuführen von Wasser in das Gefäß nach dem Spülen mit dem ersten Spülwasser, um das Gefäß mit einem zweiten Spülwasser zu füllen; und
Spülen des Endoskops mit dem zweiten Spülwasser, wobei beim Füllen des Gefäßes mit dem zweiten Spülwasser nur Wasser in das Gefäß gefüllt wird, so dass das zweite Spülwasser kein Tensid enthält, **dadurch gekennzeichnet, dass**
wenn das Gefäß mit dem ersten Spülwasser gefüllt wird, ein Tensid in das Gefäß zusätzlich zu dem Wasser eingefüllt wird, wobei es in einer solchen Menge eingestellt wird, dass eine Konzentration des Tensids in dem ersten Spülwasser innerhalb des Gefäßes geringer ist als eine Konzentration des Tensids in dem in dem Reinigungsschritt verwendeten Reinigungsmittel, und sich in der Nähe einer kritischen Mizellenkonzentration befindet.

2. Endoskop-Spülverfahren nach Anspruch 1, bei dem Wasser von einem Wasservorrat bei normaler Temperatur in zumindest sämtlichen Schritten des Spülens mit dem ersten Spülwasser verwendet wird, wohingegen teilweise oder bei sämtlichen Schritten des Spülens mit dem zweiten Spülwasser von dem Wasservorrat zugeführtes Wasser verwendet wird, nachdem dieses auf eine erhöhte Temperatur erhitzt wurde.

3. Endoskop-Spülverfahren nach Anspruch 1 oder 2, bei dem
zunächst ein Überlauf-Spülvorgang derart durchgeführt wird, dass das Endoskop mit Spülwasser gespült wird, welches teilweise von dem Behälter abgezogen wird, während das so abgezogene Spülwasser durch Frischwasserzufuhr kompensiert wird, und
anschließend eine Badspülung in der Weise durchgeführt wird, dass das Endoskop mit Frischwasser gespült wird, welches vollständig in das Gefäß geleitet wird, nachdem das Spülwasser vollständig aus dem Gefäß abgezogen wurde.

4. Endoskop-Spülverfahren nach Anspruch 3, bei dem
der Überlauf-Spülvorgang derart durchgeführt wird, dass das Endoskop mit dem ersten Spülwasser in dem Gefäß gespült wird, indem das erste Spülwasser teilweise aus dem Gefäß abgezogen wird, während das derart abgezogene erste Spülwasser durch Frischwasserzufuhr kompensiert wird, und
der Badspülvorgang in der Weise durchgeführt wird, dass das Endoskop mit dem zweiten Spülwasser gespült wird, welches durch Zufuhr von Frischwasser in das Gefäß gefüllt wird, nachdem das erste Spülwasser vollständig aus dem Behälter abgezogen wurde.

5. Endoskop-Spülverfahren nach einem der Ansprüche 1 bis 4, bei dem das Tensid in das Gefäß eingebracht wird, während das Wasser in das Gefäß geleitet wird, so dass das Gefäß mit dem ersten Spülwasser gefüllt wird.

6. Endoskop-Spülverfahren nach Anspruch 5, bei dem das Tensid kontinuierlich oder intermittierend nach Maßgabe der Zufuhr des in das Gefäß eingeleiteten Wassers eingebracht wird.

7. Endoskop-Spülverfahren nach einem der Ansprüche 1 bis 6, bei dem nur Wasser in das Gefäß geleitet wird, nachdem das erste Spülwasser vollständig aus dem Gefäß abgezogen wurde, so dass das Gefäß mit dem zweiten Spülwasser gefüllt wird.

8. Endoskop-Spülverfahren nach einem der Ansprüche 1 bis 7, bei dem das Wasser von einem Wasservorrat zugeführt wird.

9. Endoskop-Spülverfahren nach einem der Ansprüche 1 bis 8, bei dem das Wasser Leitungswasser von einem Wasserhahn ist.

10. Endoskop-Spülverfahren nach einem der Ansprüche 1 bis 9, bei dem das Tensid ein anionisches Tensid ist.

11. Endoskop-Spülverfahren nach einem der Ansprüche 1 bis 10, bei dem das Tensid ein Tensid ist, welches in einem Reinigungsmittel enthalten ist, welches zum Reinigen des Endoskops verwendet wird.

12. Endoskop-Spülverfahren nach einem der Ansprüche 1 bis 12, bei dem ein zum Reinigen des Endoskops verwendetes Reinigungsmittel zum Einstellen des Tensids in dem ersten Spülwasser verwendet wird.

## Revendications

1. Procédé de rinçage d'un endoscope placé dans un récipient inclus dans un nettoyeur d'endoscope (10) comprenant les étapes suivantes après l'achèvement d'une étape de nettoyage utilisant un détergent qui inclut un tensioactif :
le chargement d'eau dans un récipient (14a, 14b) pour remplir ledit récipient (14a, 14b) avec une première eau de rinçage ;
le rinçage dudit endoscope placé dans ledit récipient avec ladite première eau de rinçage ;
la distribution d'eau dans ledit récipient après le rinçage avec ladite première eau de rinçage pour remplir ledit récipient avec une seconde eau de rinçage ; et
le rinçage dudit endoscope avec ladite seconde eau de rinçage, où, lors du remplissage dudit récipient avec ladite seconde eau de rinçage, seule de l'eau est distribuée dans ledit récipient de sorte que ladite seconde eau de rinçage ne contient pas de tensioactif, **caractérisé en ce que**, lors du remplissage dudit récipient avec ladite première eau de rinçage, un tensioactif est chargé dans ledit récipient en plus de l'eau de manière à être ajusté à une quantité telle qu'une concentration du tensioactif dans ladite première eau de rinçage dans ledit récipient est inférieure à une concentration du tensioactif dans ledit détergent utilisé dans ladite étape de nettoyage et est au voisinage d'une concentration micellaire critique.

2. Procédé de rinçage d'un endoscope selon la revendication 1, où l'eau telle qu'elle est distribuée depuis une distribution d'eau est utilisée à la température ordinaire dans au moins toutes les étapes de rinçage avec ladite première eau de rinçage tandis que l'eau telle qu'elle est distribuée depuis ladite distribution d'eau est utilisée après avoir été chauffée à une température élevée dans tout ou partie des étapes de rinçage avec ladite seconde eau de rinçage.

3. Procédé de rinçage d'un endoscope selon la revendication 1 ou 2, où
un rinçage à débordement est d'abord réalisé de telle manière que ledit endoscope est rincé avec de l'eau de rinçage qui est partiellement évacuée dudit récipient tandis que l'eau de rinçage ainsi évacuée est compensée par une distribution fraîche d'eau, et
ensuite, un rinçage en bain est réalisé de telle manière que ledit endoscope est rincé avec une eau fraîche totalement distribuée dans ledit récipient après que ladite eau de rinçage a été totalement évacuée dudit récipient.

4. Procédé de rinçage d'un endoscope selon la revendication 3, où
ledit rinçage à débordement est réalisé de telle manière que ledit endoscope est rincé avec ladite première eau de rinçage dans ledit récipient par évacuation partielle de ladite première eau de rinçage dudit récipient tandis que la première eau de rinçage ainsi évacuée est compensée par ladite distribution fraîche d'eau, et
ledit rinçage en bain est réalisé de telle manière que ledit endoscope est rincé avec ladite seconde eau de rinçage introduite dans ledit récipient par distribution de ladite eau fraîche dans ledit récipient après que ladite première eau de rinçage a été totalement évacuée dudit récipient.

5. Procédé de rinçage d'un endoscope selon l'une quelconque des revendications 1 à 4, où ledit tensioactif est chargé dans ledit récipient tandis que ladite eau est versée dans ledit récipient, de sorte que ledit récipient est rempli avec ladite première eau de rinçage.

6. Procédé de rinçage d'un endoscope selon la revendication 5, où ledit tensioactif est chargé en continu par intermittence dans ledit récipient selon la distribution de ladite eau versée dans ledit récipient.

7. Procédé de rinçage d'un endoscope selon l'une quelconque des revendications 1 à 6, où seule ladite eau est distribuée dans ledit récipient après que ladite première eau de rinçage a été totalement évacuée dudit récipient, de sorte que ledit récipient est rempli avec ladite seconde eau de rinçage.

8. Procédé de rinçage d'un endoscope selon l'une quelconque des revendications 1 à 7, où ladite eau est distribuée depuis une distribution d'eau.

9. Procédé de rinçage d'un endoscope selon l'une quelconque des revendications 1 à 8, où ladite eau est de l'eau du robinet distribuée depuis un robinet.

10. Procédé de rinçage d'un endoscope selon l'une quelconque des revendications 1 à 9, où ledit tensioactif est un tensioactif anionique.

11. Procédé de rinçage d'un endoscope selon l'une quelconque des revendications 1 à 10, où ledit tensioactif est un tensioactif contenu par un détergent utilisé pour le nettoyage dudit endoscope.

12. Procédé de rinçage d'un endoscope selon l'une quelconque des revendications 1 à 11, où un détergent utilisé pour le nettoyage dudit endoscope est utilisé pour l'ajustement dudit tensioactif dans ladite première eau de rinçage.
